(19) Europäisches Patentamt
European Patent Office
Office européen des brevets

(11) Veröffentlichungsnummer: **0 185 618 A2**

# (12) EUROPÄISCHE PATENTANMELDUNG

(21) Anmeldenummer: **85810594.3**

(22) Anmeldetag: **13.12.85**

(51) Int. Cl.$^4$: **C 09 B 57/04**
C 07 D 403/12, C 07 D 403/14
C 08 K 5/34

(30) Priorität: **19.12.84 CH 6014/84**

(43) Veröffentlichungstag der Anmeldung:
**25.06.86 Patentblatt 86/26**

(84) Benannte Vertragsstaaten:
**CH DE FR GB IT LI**

(71) Anmelder: **CIBA-GEIGY AG**
**Klybeckstrasse 141**
**CH-4002 Basel(CH)**

(72) Erfinder: **Jost, Max**
**Rebgartenweg 20**
**CH-4104 Oberwil(CH)**

(72) Erfinder: **von der Crone, Jost, Dr.**
**La Dey**
**CH-1711 Arconciel(CH)**

## (54) Isoindoline, Verfahren zu deren Herstellung und Verwendung.

(57) Isoindoline der Formel I

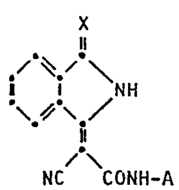

(I)

worin A für eine Gruppe der Formel II

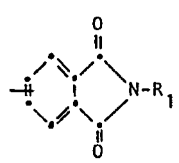

(II) steht,

worin $R_1$ –H, $C_1$–$C_4$–Alkyl oder einen unsubstituierten oder durch nicht löslichmachende Gruppen substituierten Phenylrest bedeutet, X eine Gruppe der Formel III

NC–C–CONH$R_2$ (III)

worin $R_2$ einen unsubtituierten oder durch nichtlöslichmachenda Substituenten substituierten isocyclischen oder einen heterocyclischen aromatischen Rest bedeutet, oder X ein heterocyclischer Rest, der einen ankondensierten Benzolkern aufweisen kann, ist, eignen sich zum Pigmentieren von hochmolekularem organischem Material.

EP 0 185 618 A2

Croydon Printing Company Ltd

0185618

CIBA-GEIGY AG 3-15195/-

Basel (Schweiz)

Isoindoline, Verfahren zu deren Herstellung und Verwendung

Die Erfindung betrifft neue Isoindoline enthaltend einen Phthalsäureimidrest, ein Verfahren zur Herstellung dieser Isoindoline sowie ihre Verwendung zum Pigmentieren von hochmolekularem organischem Material.

Isoindoline enthaltend einen Phthalimidoanilinrest sind bekannt und beispielsweise in der Deutschen Offenlegungsschrift Nr. 2814526 als Pigmente zum Färben von hochmolekularem organischem Material beschrieben.

Gegenstand der vorliegenden Erfindung sind Isoindoline der Formel I

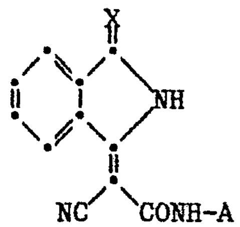

(I),

worin A für eine Gruppe der Formel II

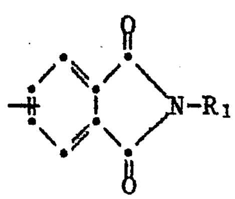

(II) steht,

worin $R_1$ -H, $C_1$-$C_4$-Alkyl oder einen unsubstituierten oder durch nicht löslichmachende Gruppen substituierten Phenylrest bedeutet, X eine Gruppe der Formel III

$NC-\overset{H_2}{C}-CONHR_2$ (III)

worin $R_2$ einen unsubstituierten oder durch nichtlöslichmachende Substituenten substituierten isocyclischen oder einen heterocyclischen aromatischen Rest bedeutet, oder X ein heterocyclischer Rest, der einen ankondensierten Benzolkern aufweisen kann, ist.

Bedeutet $R_1$ $C_1$-$C_4$-Alkyl, so handelt es sich dabei beispielsweise um Methyl, Aethyl, n-Propyl oder n-Butyl, insbesonders aber um Methyl.

Unter nicht löslichmachenden Substituenten für den Phenylrest als $R_1$ versteht man solche, die weder in Wasser noch in organischen Lösungsmitteln eine Lösung des Pigmentes bewirken, also beispielsweise Halogenatome, Alkyl- oder Alkoxygruppen mit 1 - 6 C-Atomen, Nitro-, Trifluormethyl-, Carbamyl-, Ureido-, Sulfamyl- oder Cyangruppen, Alkoxycarbonyl-, Alkylcarbonyl-, N-Alkylcarbamyl-, N-Alkylureido- oder Alkanoylaminogruppen mit 2 -6 C-Atomen, Alkylsulfonyl- oder N-Alkylsulfamylgruppen mit 1 - 6 C-Atomen, Aryloxycarbonyl-, Arylcarbonyl-, Aroylamino-, Arylsulfonyl-, N-Arylcarbamyl-, N-Arylsulfamyl-, Aryl-, N-Arylureido- oder Arylazogruppen.

Bedeutet X einen heterocyclischen Rest, der einen ankondensierten Benzolrest aufweisen kann, so handelt es sich dabei beispielsweise um einen 5- oder 6-gliedrigen Rest der Formeln

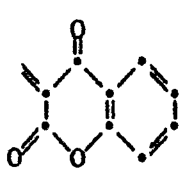 , 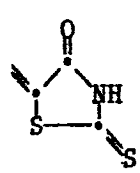 , 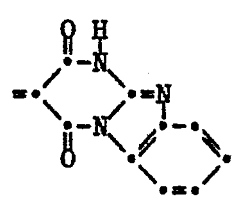 ,

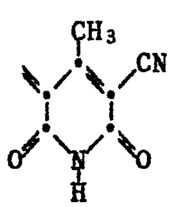 , 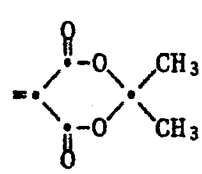 , 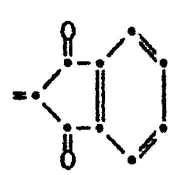 oder insbesondere

der Formeln 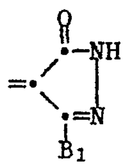 , 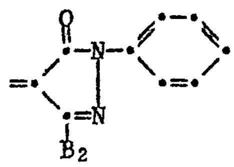 oder bevorzugt

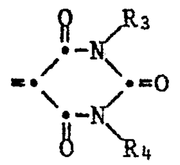 ,

wobei $B_1$ durch Chlor, Methyl oder Methoxy substituiertes oder insbesondere unsubstituiertes Phenyl, $B_2$ Methyl, Phenyl, oder insbesondere Carbamyl oder Acetylamino und $R_3$ und $R_4$ unabhängig voneinander -H, $C_1$-$C_4$-Alkyl, unsubstituiertes oder durch Halogen, $C_1$-$C_4$-Alkyl, $C_1$-$C_4$-Alkoxy oder Phenoxy substituiertes Phenyl bedeuten.

$C_1$-$C_4$-Alkyl und $C_1$-$C_4$-Alkoxy als Phenylsubstituenten für $R_3$ und $R_4$ sind beispielsweise Methyl-, Aethyl-, n-Propyl-, n-Butylgruppen bzw. Methoxy-, Aethoxy-, n-Propoxy- und n-Butoxygruppen.

Bedeutet $R_2$ einen isocyclischen aromatischen Rest, so handelt es sich dabei beispielsweise um Phenyl oder Naphthyl, insbesonders aber um durch nicht löslichmachende Gruppen substituiertes Phenyl. Unter nicht löslichmachenden Gruppen versteht man diejenigen Gruppen, die für die Definition von $R_1$ bereits oben angegeben worden sind.

Stellt $R_2$ einen heterocyclischen aromatischen Rest dar, so leitet sich dieser beispielsweise von folgenden heterocyclischen Aminen ab:

5-Amino-benzimidazolon, 5-Amino-1-methyl-benzimidazolon, 5-Amino-1-n-butyl-benzimidazolon, 5-Amino-1-phenyl-benzimidazolon, 5-Amino-1-p-chlorphenyl-benzimidazolon, 5-Amino-1-p-methylphenyl-benzimidazolon, 5-Amino-1-p-methoxyphenyl-benzimidazolon, 5-Amino-6-chlor-benzimidazolon, 5-Amino-6-brom-benzimidazolon, 5-Amino-6-methyl-benzimidazolon, 5-Amino-6-methoxy-benzimidazolon, 6-Aminobenzoxazolon, 5-Aminobenzoxazolon, 5-Amino-7-chlor-benzoxazolon,

0185618

6-Amino-7-chlor-benzoxazolon, 6-Amino-5-chlor-benzoxazolon, 6-Amino-5-methyl-benzoxazolon, 6-Amino-5-chlor-benzthiazolon, 6-Amino-5-methyl-benzthiazolon, 6-Aminochinazolon-4, 6-Amino-2-methyl-chinazolon-4, 6-Amino-2-methoxy-chinazolon-4, 6-Amino-7-chlor-2-methyl-chinazolon-4, 7-Aminochinazolon-4, 2-(4'-Aminophenyl)-chinazolon-4, 2-(3'-Aminophenyl)-chinazolon-4, 2-(4'-Amino-3'-methoxyphenyl)-chinazolon-4, 2-(4'-Amino-3'-chlorphenyl)-chinazolon-4, 2-(3'-Amino-4'-methylphenyl)-chinazolon-4, 6-Amino-2,4-dihydroxychinazolin, 7-Amino-phenmorpholon-3, 6-Amino-phenmorpholon-3, 7-Amino-6-chlor-phenmorpholon-3, 7-Amino-6-methyl-phenmorpholon-3, 7-Amino-6-methoxy-phenmorpholon-3, 6-Amino-chinolon-2, 6-Amino-4-methyl-chinolon-2, 7-Amino-4-methyl-chinolon-2, 7-Amino-4,6-dimethyl-chinolon-2, 6-Amino-7-chlor-4-methyl-chinolon-2, 7-Amino-4-methyl-6-methoxy-chinolon-2, 6-Amino-1,3-dihydroxy-isochinolin, 6-Amino-2,4-dihydroxy-chinolin, 6-Amino-2,3-dihydroxychinoxalin, 4-Amino-phthalsäureimid, 3- oder 4-Aminoacridon und 3- oder 4-Amino-naphthostyryl, ferner 2-Aminothiazol-1,3, 3-Amino-triazol-1,2,4, 3-Amino-5-phenyl-triazol-1,2,4, 2-Aminobenzimidazol, 2-Amino-3-N-methyl-benzimidazol, 2-Aminobenzthiazol, 2-Aminobenzoxazol, 2-Amino-chinazolon-4 und 2-Amino-chinoxalinon-3. Bevorzugt ist der Rest, der sich von 3-Amino-triazol-1,2,4 ableitet.

Bei den oben angegebenen Gruppen, welche Halogen darstellen, handelt es sich um Chlor, Brom, Jod und Fluor, insbesonders aber um Chlor und Brom, ganz bevorzugt aber um Chlor.

Bevorzugt sind Isoindoline der Formel IV

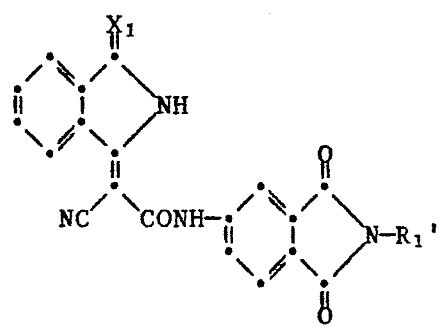

(IV),

worin $R_1'$ -H oder Methyl bedeutet und $X_1$ für einen heterocyclischen aromatischen Rest, ausgewählt aus der Gruppe bestehend aus den Resten der Formeln

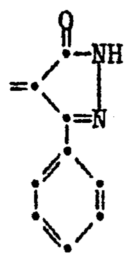 , 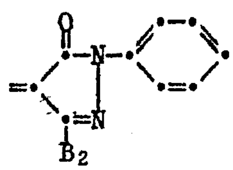 oder 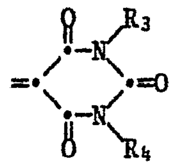 steht,

worin $B_2$ eine Gruppe $-CONH_2$ oder $-NHCOCH_3$ ist und $R_3$ und $R_4$ unabhängig voneinander -H, $C_1$-$C_4$-Alkyl, unsubstituiertes oder durch Halogen, $C_1$-$C_4$-Alkyl, $C_1$-$C_4$-Alkoxy oder Phenoxy substituiertes Phenyl bedeuten, oder $X_1$ für den Rest der Formel

$NC-\overset{\|}{C}-CONHR_5$

steht, worin $R_5$ unsubstituiertes oder durch eine oder zwei nichtlöslich machende Substituenten substituiertes Phenyl oder eine Gruppe

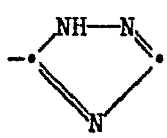

bedeutet.

Besonders bevorzugt sind Isoindoline der Formel V

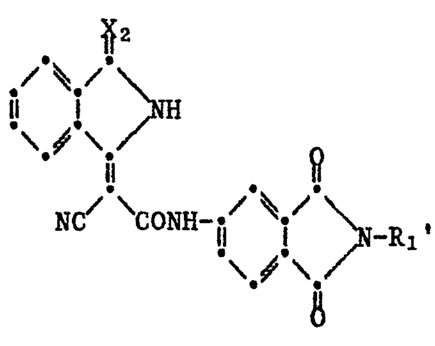

(V), worin

$X_2$ für einen Rest der Formeln

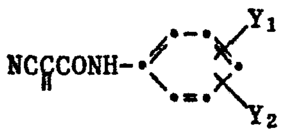 oder 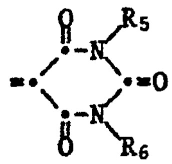 steht, und

$R_1$' -H oder Methyl bedeutet, $Y_1$ und $Y_2$ unabhängig voneinander ein H- oder Chloratom, eine Alkyl-, Alkoxy-, Trifluormethyl-, Carbamyl-, N-Methylcarbamyl-, Methoxycarbonyl-, Aethoxycarbonyl-, Acetylamino-, unsubstituierte oder durch ein oder zwei Chloratome, Methyl- oder Methoxygruppen substituierte Benzoylaminogruppe und $R_5$ und $R_6$ unabhängig voneinander -H, $-CH_3$ oder unsubstituiertes oder durch -Cl, $-CH_3$ oder $-OCH_3$ substituiertes Phenyl bedeuten. Besonders bevorzugt sind $Y_1$ und $Y_2$ unabhängig voneinander H, Cl, $CH_3$ oder $OCH_3$.

Zu den erfindungsgemässen Verbindungen der Formel I gelangt man, wenn man das Diiminoisoindolin der Formel VI

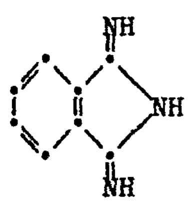 (VI)

in beliebiger Reihenfolge mit je einem Mol eines Cyanacetylaminoderivates der Formel VII

$CN$
$CH_2CONH-A$ (VII)

und einer Verbindung der Formel $XH_2$, worin A und X die angegebene Bedeutung haben, kondensiert.

Als Verbindungen der Formel VII werden insbesonders Verbindungen der Formel

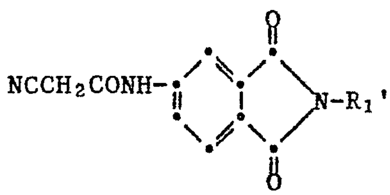

verwendet, worin $R_1'$ die oben angegebene Bedeutung hat.

Als Beispiele von Cyanacetylaminoderivaten der Formel VII seien genannt:

4-Cyanacetylaminophthalimid
3-Cyanacetylaminophthalimid
4-Cyanacetylaminophthal-N-methylimid
3-Cyanacetylaminophthal-N-methylimid
4-Cyanacetylaminophthal-N-äthylimid
4-Cyanacetylaminophthal-N-phenylimid
3-Cyanacetylaminophthal-N-phenylimid
4-Cyanacetylaminophthal-N-parachlorphenylimid
4-Cyanacetylaminophthal-N-paramethylphenylimid
4-Cyanacetylaminophthal-N-paramethoxyphenylimid
4-Cyanacetylaminophthal-N-4'-acetylaminophenylimid
4-Cyanacetylaminophthal-N-4'-methoxycarbonylphenylimid
4-Cyanacetylaminophthal-N-2',5'-dichlorphenylimid
4-Cyanacetylaminophthal-N-2'-methoxyphenylimid

Als Beispiele von Verbindungen der Formel $XH_2$ seien genannt:

Cyanessigsäureanilid
Cyanessigsäure-2'-, 3'- oder 4'-chloranilid

Cyanessigsäure-2',4'-dichloranilid
Cyanessigsäure-2',5'-dichloranilid
Cyanessigsäure-2',3'-dichloranilid
Cyanessigsäure-3',4'-dichloranilid
Cyanessigsäure-3',5'-dichloranilid
Cyanessigsäure-2',4',5'-trichloranilid
Cyanessigsäure-3',4',5'-trichloranilid
Cyanessigsäure-4'-bromanilid
Cyanessigsäure-3',4'-dibromanilid
Cyanessigsäure-3'-chlor-4'-bromanilid
Cyanessigsäure-3'-brom-4'-chloranilid
Cyanessigsäure-2'-, 3'- oder 4'-methylanilid
Cyanessigsäure-2',4'-dimethylanilid
Cyanessigsäure-2',4',6'-trimethylanilid
Cyanessigsäure-2'-, 3'- oder 4'-methoxyanilid
Cyanessigsäure-4'-acetylaminoanilid
Cyanessigsäure-4'-benzoylaminoanilid
Cyanessigsäure-4'-p-chlor-benzoylamino-anilid
Cyanessigsäure-4'-phthalimido-anilid
Cyanessigsäure-2',5'-dichlor-4'-benzoylamino-anilid
Cyanessigsäure-2',5'-dichlor-4'-p-chlorbenzoylamino-anilid
Cyanessigsäure-2',5'-dichlor-4'-methoxy-anilid
Cyanessigsäure-2',5'-dimethoxy-4'-benzoylamino-anilid
Cyanessigsäure-4'-nitroanilid
Cyanessigsäure-3'-chlor-4'-methyl-anilid
Cyanessigsäure-2'-, 3'- oder 4'-methoxycarbonyl-anilid
Cyanessigsäure-2'-, 3'- oder 4'-äthoxycarbonyl-anilid
Cyanessigsäure-3'-trifluormethyl-anilid
Cyanessigsäure-2',5'-diäthoxy-4'-benzoylamino-anilid
Cyanessigsäure-2',5'-dimethoxy-4'-chloranilid
Cyanessigsäure-4'-carbamyl-anilid
Cyanessigsäure-3'-chlor-4'-carbamyl-anilid und
Cyanessigsäure-2'-methyl-4'-carbamyl-anilid,

sowie die Cyanacetylverbindungen folgender heterocyclischer aromatischer Amine:

5-Amino-benzimidazolon
5-Amino-1-methyl-benzimidazolon
5-Amino-6-chlor-benzimidazolon
5-Amino-6-methyl-benzimidazolon
6-Amino-chinazolon-4
6-Amino-2-methyl-chinazolon-4
7-Amino-chinazolon
2-(4'-Aminophenyl)-chinazolon-4
6-Amino-2,4-dihydroxychinazolin
7-Amino-phenmorpholon-3
6-Amino-phenmorpholon-3
7-Amino-6-methyl-phenmorpholon-3
6-Amino-chinolon-2
7-Amino-4-methyl-chinolon-2
6-Amino-7-chlor-4-methyl-chinolon-2 und
7-Amino-4,8-dimethyl-chinolon-2.

Es handelt sich dabei um bekannte Verbindungen, die durch Erhitzen von Cyanessigester mit den entsprechenden Aminen erhalten werden.

Weitere Beispiele von Verbindungen der Formel $XH_2$ sind:

Barbitursäure
1,3-Dimethyl-barbitursäure
1,3-Diaethyl-barbitursäure
1-Methyl-3-phenyl-barbitursäure
1-Phenyl-barbitursäure
1-p-Chlorphenylbarbitursäure
1-p-Methoxyphenylbarbitursäure
1-Methyl-barbitursäure
1-Phenyl-3-acetylamino-pyrazolon(5)
1-Phenyl-3-carbamyl-pyrazolon(5)
3-Phenyl-pyrazolon(5)
2,4-Dihydroxychinolin

Die Herstellung der Verbindungen der Formel I erfolgt vorzugsweise durch Monokondensation des Diiminoisoindolins der Formel VI mit einer Verbindung der Formel $XH_2$ und anschliessende Umsetzung des so erhaltenen Monokondensationsprodukts mit einer Verbindung der Formel VII.

Die Kondensation des Diiminoisoindolins mit der Verbindung der Formel $XH_2$ und der Verbindung der Formel VII erfolgt vorzugsweise in einem organischen Lösungsmittel, beispielsweise einem aliphatischen Alkohol mit 1-4 C-Atomen, wie Methanol, Aethanol, Isopropanol, n-Butanol, ferner Glykolen oder Glykoläthern wie Aethylenglykolmonomethyl- oder -monoäthyläther, offenkettigen oder cyclischen Amiden, wie Dimethylformamid, Dimethylacetamid oder N-Methylpyrrolidon, einem halogenierten Benzol, wie Monochlorbenzol, Di- oder Trichlorbenzole, Nitrobenzol oder einer aliphatischen Mono- oder Dicarbonsäure, wie Ameisensäure, Essigsäure, Propionsäure, Mono- oder Dichloressigsäure, Fumarsäure, Milchsäure oder Weinsäure, wobei auch Mischungen der genannten Lösungsmittel verwendet werden können. Die Umsetzung zu den Monokondensationsprodukten erfolgt vorteilhaft in neutralen organischen Lösungsmitteln, beispielsweise einem aliphatischen Alkohol mit 1-4 C-Atomen, wie Methanol, Aethanol, Isopropanol oder n-Butanol, wobei ein Ueberschuss an Diiminoisoindolin verwendet wird. Falls nötig, können sie isoliert und gereinigt werden. Der Ersatz der ersten Iminogruppe des Diiminoisoindolins erfolgt in der Regel schon bei Temperaturen unter 100°C. Der Austausch der zweiten Iminogruppe hingegen erfordert Temperaturen zwischen 100-200°C.

Die erhaltenen Produkte fallen zumeist schon in der Hitze aus und können durch Abfiltrieren und gegebenenfalls durch Waschen mit organischen Lösungsmitteln in reiner Form isoliert werden.

Die erfindungsgemässen Isoindoline der Formel I stellen wertvolle Pigmente dar, welche im allgemeinen eine gute Textur besitzen und meistens als Rohprodukte verwendet werden können. Falls nötig oder

erwünscht, kann man die Rohprodukte durch Mahlen oder Kneten in eine feindisperse Form überführen. Dabei werden zweckmässig Mahlhilfsmittel, wie Glas-, Kunststoff-, Stahl- oder Metallmahlkörper, anorganische und/oder organische Salze in Gegenwart oder Abwesenheit organischer Lösungsmittel verwendet. Nach dem Mahlen werden Hilfsmittel wie üblich entfernt, lösliche anorganische Salze z.B. mit Wasser und wasserunlösliche organische Hilfsmittel beispielsweise durch Wasserdampfdestillation. Auch durch Behandeln der Rohpigmente mit organischen Lösungsmitteln kann oft eine Verbesserung der Pigmenteigenschaften erreicht werden.

Ferner können die Rohpigmente durch Umfällen aus Schwefelsäure oder Hydrolyse eines in organischem Medium erzeugten Alkalimetallsalzes in eine feine Pigmentform übergeführt werden.

Die erfindungsgemässen Verbindungen eignen sich als Pigmente zum Färben von hochmolekularem organischem Material natürlicher oder künstlicher Herkunft. Es kann sich z.B. um Naturharze, trocknende Oele oder Kautschuk handeln. Es kann sich aber auch um abgewandelte Naturstoffe handeln, beispielsweise um Chlorkautschuk, um ölmodifizierte Alkydharze oder um Cellulosederivate, wie Viskose, Acetylcellulose und Nitrocellulose, und besonders um vollsynthetische organische Polyplaste, das heisst um Kunststoffe, die durch Polymerisation, Polykondensation und Polyaddition hergestellt sind. Aus der Klasse dieser Kunststoffe seien besonders folgende genannt: Polyäthylen, Polypropylen, Polyisobutylen, Polystyrol, Polyvinylchlorid, Polyvinylacetat, Polyacrylnitril, Polyacrylsäure- und Polymethacrylsäureester oder Polybutadien, sowie Kopolymerisate der erwähnten Monomeren, insbesonders ABS oder EVA; Polyester, insbesondere hochmolekulare Ester aromatischer Polycarbonsäuren mit polyfunktionellen Alkoholen, Polyamide; die Kondensationsprodukte von Formaldehyd mit Phenolen, die sogenannten Phenoplaste, und die Kondensationsprodukte von Formaldehyd mit Harnstoff, Thioharnstoff und Melamin, die sogenannten Aminoplaste; die als Lackharze verwendeten Polyester, und zwar sowohl gesättigte, wie z.B. Alkydharze, als auch ungesättigte, wie beispielsweise Maleinatharze, und ferner

die unter dem Namen "Epoxyharze" bekannten Polyadditions- bzw. Polykondensationsprodukte von Epichlorhydrin mit Diolen oder Polyphenolen; ferner die sogenannten Thermoplasten, dh. die nicht härtbaren Polyplaste. Es sei betont, dass nicht nur die einheitlichen Verbindungen, sondern auch Gemische von Polyplasten, sowie Mischkondensate und Mischpolymerisate, wie z.B. solche auf Basis von Butadien, erfindungsgemäss pigmentiert werden können.

Die mit den erfindungsgemässen Verbindungen zu pigmentierenden hochmolekularen organischen Materialien können auch im polymerisierten Zustand in gelöster Form als Filmbildnern oder Bindemitteln für Lacke oder Druckfarben vorliegen, wie z.B. Leinölfirnis, Nitrocellulose, Alkydharze, Melaminharze und Harnstoff-Formaldehydharze oder Acrylharze.

Die Pigmentierung der hochmolekularen, organischen Substanzen mit den Verbindungen der Formel I erfolgt beispielsweise derart, dass man ein solches Produkt gegebenenfalls in Form von Masterbatches, diesen Substraten unter Verwendung von Walzwerken, Misch- oder Mahlapparaturen zumischt. Das pigmentierte Material wird hierauf nach an sich bekannten Verfahren wie Kalandrieren, Pressen, Strangpressen, Streichen, Giessen, Spinnen oder durch Spritzguss in die gewünschte endgültige Form gebracht. Oft ist es erwünscht, zur Herstellung von nicht starren Formlingen oder zur Verringerung ihrer Sprödigkeit den hochmolekularen Verbindungen vor der Verformung sogenannte Weichmacher einzuverleiben. Als solche können z.B. Ester der Phosphorsäure, Phthalsäure oder Sebacinsäure dienen. Die Weichmacher können im erfindungsgemässen Verfahren vor oder nach der Einverleibung des Pigmentfarbstoffes in die Polyplasten eingearbeitet werden. Es ist ferner möglich, zwecks Erzielung verschiedener Farbtöne den hochmolekularen, organischen Stoffen neben den Verbindungen der Formel I noch Füllstoffe bzw. andere farbgebende Bestandteile wie Weiss-, Bunt- oder Schwarzpigmente in beliebigen Mengen zuzufügen.

Zum Pigmentieren von Lacken und Druckfarben werden die hochmolekularen organischen Materialien und die Verbindungen der Formel I gegebenenfalls zusammen mit Zusatzstoffen wie Füllmitteln, anderen Pigmenten, Siccativen oder Weichmachern, in einem gemeinsamen organischen Lösungsmittel oder Lösungsmittelgemisch fein dispergiert bzw. gelöst. Man kann dabei so verfahren, dass man die einzelnen Komponenten für sich oder auch mehrere gemeinsam dispergiert bzw. löst, und erst hierauf alle Komponenten zusammenbringt.

Die pigmentierten hochmolekularen organischen Materialien enthalten im allgemeinen Mengen von 0,001 bis 30 Gew.-% Pigment, bezogen auf den zu pigmentierenden hochmolekularen organischen Stoff, Polyplasten und Lacke vorzugsweise 0,1 bis 5 %, Druckfarben vorzugsweise 10 bis 30 %. Die zu wählende Menge an Pigment richtet sich in erster Linie nach der gewünschten Farbstärke, ferner nach der Schichtdicke des Formlings und schliesslich gegebenenfalls auch nach dem Gehalt an Weisspigment im Polyplast.

Die Verbindungen der Formel I weisen in den hochmolekularen organischen Substanzen sehr echte reine, gelbe bis rote Farbtöne auf und zeichnen sich in der Applikation durch gute Farbstärke, Licht-, Wetter-, Migrations-, Ueberlackier-, Lösungsmittel- und Hitzebeständigkeit aus. Ferner besitzen sie bei optimaler Korngrösse ein hohes Deckvermögen in der Applikation.

Die folgenden Beispiele veranschaulichen die Erfindung.

Beispiel 1: 3,22 g 1-(Cyan-3'-chlorphenylcarbamylmethylen)-3-imino-isoindolin und 2,7 g 4-Cyanacetylaminophthalsäure-N-methylimid werden in einem Gemisch von 50 g o-Dichlorbenzol und 20 g Essigsäure 4 Stunden bei 125°C gerührt. Das ausgefallene Produkt wird abfiltriert und mit kaltem o-Dichlorbenzol dann kaltem Methanol gewaschen. Zur Reinigung wird das noch methanolfeuchte Rohprodukt während zwei Stunden mit 60 ml Dimethylformamid bei 120°C verrührt, darauf das Produkt bei 100°C abfiltriert, mit kaltem Dimethylformamid und anschliessend kaltem Methanol gewaschen und getrocknet. Durch Mahlung beispielsweise in Diäthylenglykoldiäthyläther unter Verwendung von Natriumchlorid als Mahlkörper enthält man einen Pigmentfarbstoff der Formel

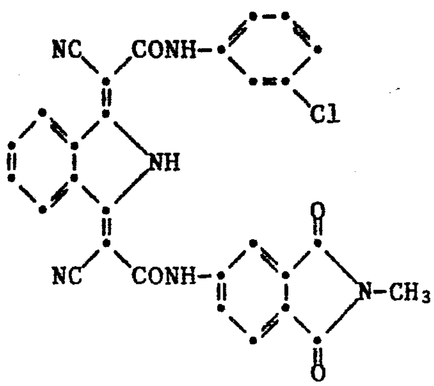

,

welcher PVC und Lacke in farbstarken gelben Tönen mit guter Migrations- und vorzüglicher Licht- und Wetterbeständigkeit färbt.

Beispiele 2-12: Auf analoge Weise wie in Beispiel 1 beschrieben werden die in Tabelle I aufgeführten Produkte erhalten. Sie zeichnen sich in der Applikation durch eine gute Farbstärke und Migrationsbeständigkeit aus, besitzen vorzügliche Ueberlackier-, Licht- und Wetterbeständigkeit und zeigen in mehreren Fällen ein hohes Deckvermögen.

0185618

Tabelle I

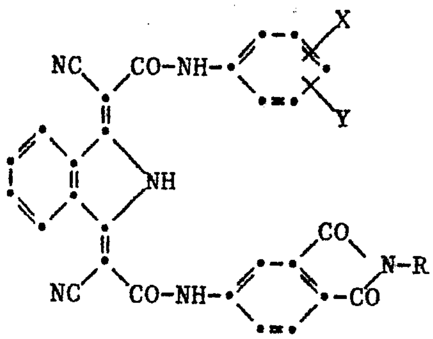

| Beispiel Nr. | X | Y | R | Nuance in PVC (0,2 %) |
|---|---|---|---|---|
| 2 | 3-Cl | -H | -H | braunorange |
| 3 | 4-Cl | -H | -H | braunorange |
| 4 | 4-Cl | -H | $-CH_3$ | gelb |
| 5 | -H | -H | $-CH_3$ | rotstichig gelb |
| 6 | -H | -H | -H | braunorange |
| 7 | $4-OCH_3$ | -H | -H | braun |
| 8 | $4-OCH_3$ | -H | $-CH_3$ | braun |
| 9 | 3-Cl | 4-Cl | $-CH_3$ | gelb |
| 10 | 3-Cl | 4-Cl | -H | gelb |
| 11 | 3-Cl | 5-Cl | -H | rotstichig gelb |
| 12 | 3-Cl | 5-Cl | $-CH_3$ | gelb |

<u>Beispiel 13:</u> 3,7 g 1-Cyan-N-methylphthalimid-(4'-)ylcarbamylmethylen-3-iminoisoindolin und 2,1 g N-4'-Methoxyphenylbarbitursäure werden in 90 g Essigsäure während 4 Stunden bei 110°C verrührt. Das entstandene Produkt wird heiss abfiltriert und mit kalter Essigsäure dann kaltem Aethanol gewaschen. Das lösungsmittelfeuchte Produkt wird mit 60 g Dimethylformamid während 2 Stunden bei 120°C verrührt, das entstandene Produkt abfiltriert, mit kaltem Dimethylformamid dann kaltem Aethanol gewaschen und bei 80°C unter Vakuum getrocknet. Man erhält 4,4 g eines Produktes der Formel

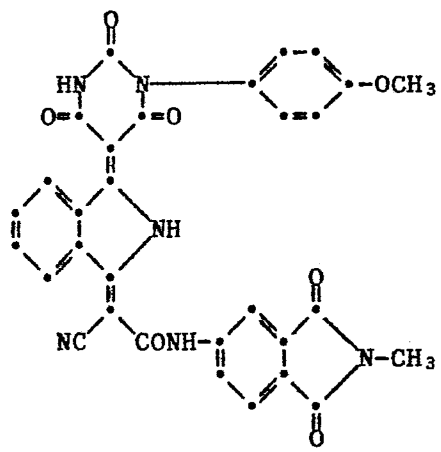

,

das nach Ueberführung in eine feinteilige Form in Kunststoffen und Lacken rotstichig gelbe Färbungen mit vorzüglicher Migrations-, Licht-, Wetter- und Ueberlackierbeständigkeit ergibt.

<u>Beispiel 14-24:</u> In analoger, gemäss Beispiel 13 beschriebener Weise werden die in Tabelle II angeführten Produkte erhalten:

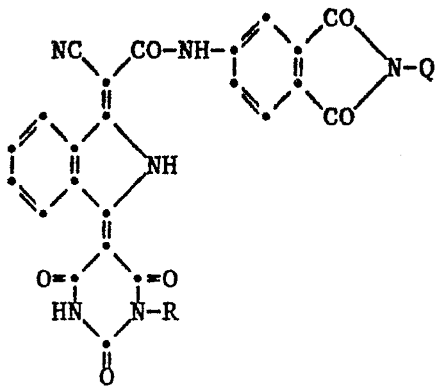

<u>Tabelle II</u>

| Beispiel | R | Q | Nuance in PVC (0,2 % Pigment) |
|---|---|---|---|
| 14 | -H | $-CH_3$ | gelb |
| 15 | Phenyl | $-CH_3$ | gelb |
| 16 | -H | -H | rotstichig gelb |
| 17 | $-CH_3$ | -H | gelb |
| 18 | Phenyl | -H | gelb |
| 19 | p-Methoxyphenyl | -H | rotstichig gelb |
| 20 | 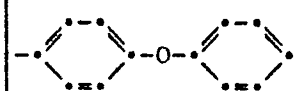 | -H | rotstichig gelb |
| 21 | 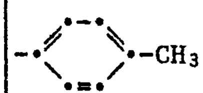 | -H | gelb |
| 22 | 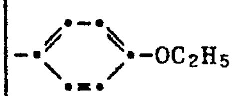 | -H | orange |
| 23 | 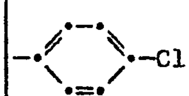 | H | gelb |
| 24 | $-CH_3$ | $-CH_3$ | gelb |

Beispiel 25: 3,57 g 1-Cyan-phthalimid-(4'-)ylcarbamylmethylen-3-iminoisoindolin und 3,05 g N-3'-Chlorphenyl-N'-methylbarbitursäure werden in 60 g Essigsäure 4 Stunden bei Siedetemperatur gerührt. Die erhaltene, gelborange Suspension wird heiss filtriert. Das isolierte Produkt wird nach Waschen mit Essigsäure und Methanol mit 60 g Dimethylformamid 2 Stunden bei 120°C verrührt, wiederum heiss abfiltriert, dann mit Dimethylformamid und anschliessend mit Methanol gewaschen und getrocknet. Nach Ueberführung in eine feindisperse Form färbt das erhaltene Pigment der Formel

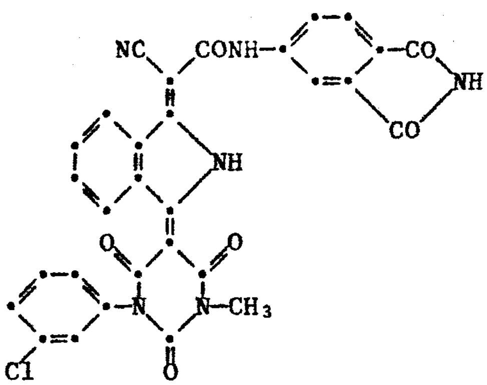

PVC und Lacke in reinen gelben Tönen.

Beispiele 26-31: In analoger Weise wie in Beispiel 25 beschrieben, werden die in Tabelle III angeführten Produkte der nachstehenden Formel (X) erhalten:

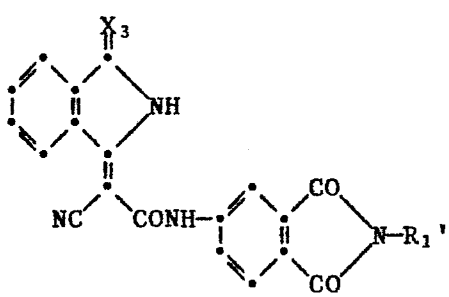

(X)

Tabelle III

| Beispiel | $X_3$ | $R_1'$ | Nuance in PVC (0,2 % Pigment) |
|---|---|---|---|
| 26 | 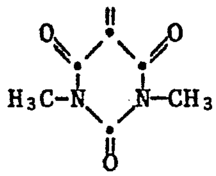 | —H | gelb |
| 27 | 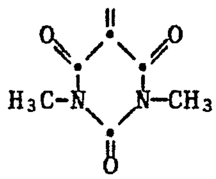 | $-CH_3$ | gelb, gut deckend |
| 28 | 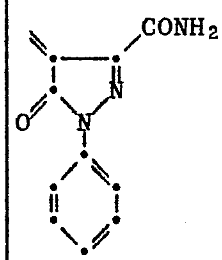 | $-CH_3$ | braun |
| 29 | 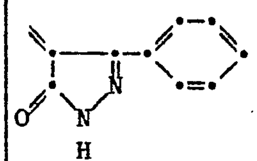 | —H | grau |
| 30 | 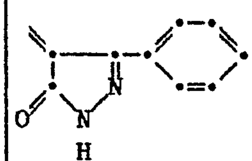 | $-CH_3$ | grau |
| 31 | 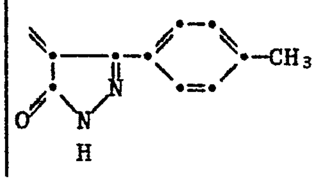 | —H | graubraun |

Beispiel 32: 3,7 g 1-Cyan-N-methylphthalimid-(4'-)ylcarbamylmethylen-3-iminoisoindolin und 1,93 g 2,4-Dihydroxychinolin werden mit 105 g o-Dichlorbenzol und 12,5 g Benzoylchlorid während 3 Stunden bei 120-125°C verrührt. Nach Zugabe von 40 g Essigsäure wird die erhaltene Suspension weitere 3 Stunden bei 115°C gehalten. Das entstandene Produkt wird abfiltriert und mit o-Dichlorbenzol und anschliessend mit Aethanol gewaschen. Das Rohprodukt wird mit 120 g Dimethylformamid 1 Stunde bei 120°C verrührt, heiss abfiltriert, mit Dimethylformamid und anschliessend mit Methanol gewaschen und getrocknet. Nach Ueberführung in eine feindisperse Form färbt das Produkt der Formel

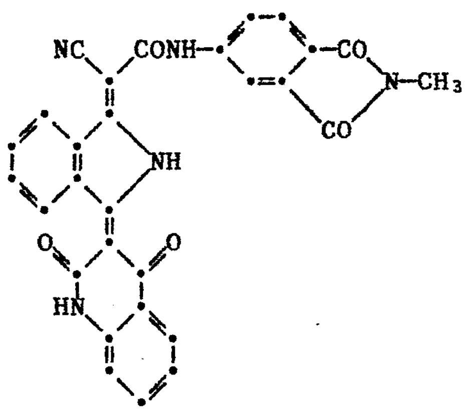

PVC und Lacke in braunorangen Tönen.

Beispiel 33: 7,3 g 1-Cyan-phthalimid-(4'-)ylcarbamylmethylen-3-iminoisoindolin und 3,2 g Barbitursäure werden in 160 g Essigsäure während 4 Stunden bei 110°C verrührt. Das ausgefallene Pigment wird heiss abfiltriert, mit Essigsäure sowie Methanol gewaschen und getrocknet. Man erhält 9.1 g (97 % d.Th.) eines Pigmentes, das nach Ueberführung in eine feinteilige Form Kunststoffe und Lacke in reinem grünstichig gelbem Ton mit sehr guter Licht- und Migrationsechtheit färbt.
Wird das erhaltene Pigment in Dimethylformamid zwei Stunden bei 120°C behandelt und anschliessend mittels Scheibenrührer in einem Natriumchlorid-Diäthylenglykoldiäthyläther-Gemisch gemahlen, so

erhält man das in Beispiel 16 beschriebene rotstichig gelb färbende Pigment. Die beiden Pigmentformen zeigen unterschiedliche Röntgenpulverdiagramme.

Beispiel 34: 2,9 g 1-Cyan-phenylcarbamylmethylen-3-iminoisoindolin und 3,5 g 4-Cyanacetylamino-N-phenylphthalimid werden in einem Gemisch von 25 g Essigsäure und 65 g o-Dichlorbenzol vier Stunden bei 120-125°C verrührt. Das ausgefallene Pigment wird abfiltriert, mit o-Dichlorbenzol sowie Methanol gewaschen und getrocknet. Das Pigment der Formel:

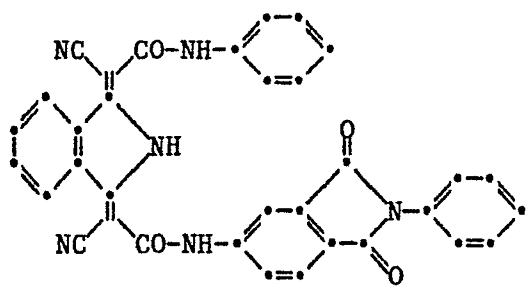

färbt Kunststoffe und Lacke in rotstichig gelbem Ton mit sehr guten Echtheitseigenschaften.

Beispiel 35: 3,22 g 1-Cyan-4'-chlorphenylcarbamylmethylen-3-iminoisoindolin und 3,5 g 4-Cyanacetylamino-N-phenylphthalimid werden in 60 g Essigsäure vier Stunden bei 110°C verrührt. Das ausgefallene Pigment wird heiss abfiltriert und mit Essigsäure sowie Aethanol gewaschen. Das alkoholfeuchte Produkt wird mit 100 g Dimethylformamid zwei Stunden bei 120°C verrührt, heiss abfiltriert, mit Dimethylformamid sowie Methanol gewaschen und getrocknet. Das erhaltene Pigment färbt Kunststoffe und Lacke in rotstichig gelbem Ton mit sehr guten Echtheitseigenschaften.

Beispiel 36: Die Mischung von 3,0 g 1-Cyan-N-phenylphthalimid-(4')ylcarbamylmethylen-3-iminoisoindolin, 1,3 g Barbitursäure und 70 g Essigsäure wird vier Stunden bei 114°C verrührt. Das ausgefallene Pigment wird heiss abfiltriert, mit Essigsäure sowie

Aethanol gewaschen und getrocknet. Man erhält 3,7 g eines Pigmentes, das nach Ueberführung in eine feinteilige Form Kunststoffe und Lacke in gelbem Ton mit guten Echtheitseigenschaften färbt.

Beispiel 37: 3,22 g 1-Cyan-4'-chlorphenylcarbamylmethylen-3-imino-isoindolin und 3,5 g 3-Cyanacetylamino-N-phenylphtalimid werden in einem Gemisch von 65 g o-Dichlorbenzol und 25 g Essigsäure vier Stunden bei 120°C gerührt. Das entstandene Pigment wird heiss abfiltriert, mit o-Dichlorbenzol sowie Aethanol gewaschen und darauf mit 80 g Dimethylformamid während zwei Stunden bei 120°C verrührt. Das Pigment wird heiss abfiltriert, mit Dimethylformamid sowie Methanol gewaschen und getrocknet. Nach Ueberführung in eine feinteilige Form erhält man ein Kunststoffe und Lacke in gelbem Ton mit sehr guten Echtheitseigenschaften färbendes Pigment der Formel:

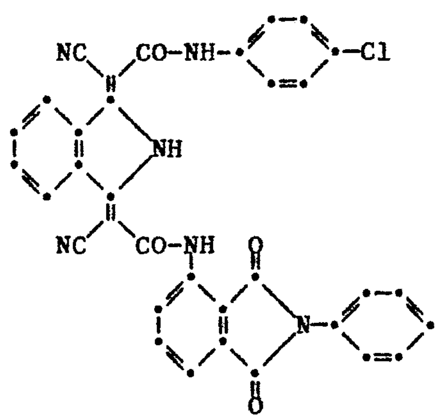

Beispiel 38: Aus 1-Cyan-3',4'-dichlorphenylcarbamylmethylen-3-iminoisoindolin und 3-Cyanacetylamino-N-phenylphthalimid erhält man analog Beispiel 37 ein Pigment, das Kunststoffe und Lacke in sehr echtem gelben Ton färbt.

Beispiel 39: 2,8 g 1-Cyan-N-phenylphthalimid-(4')ylcarbamylmethylen-3-iminoisoindolin und 1,8 g 1-Phenyl-3-acetylaminopyrazolon(5) werden in 70 g Essigsäure 15 Stunden bei 110°C verrührt. Das in üblicher Weise abgetrennte Produkt wird in 70 g Dimethylformamid zwei Stunden bei 120°C verrührt. Nach Abkühlen auf 70°C wird das dunkle Pigment abfiltriert, mit Dimethylformamid sowie Methanol

gewaschen und getrocknet. Nach Ueberführen in eine feinteilige Form erhält man ein Pigment, das Kunststoffe und Lacke in braunem Ton mit sehr guten Echtheitseigenschaften färbt.

Beispiel 40: 2,32 g 1-Cyan-phenylcarbamylmethylen-3-iminoisoindolin und 2,3 g 3-Cyanacetylaminophthalimid werden in einem Gemisch von 65 g o-Dichlorbenzol und 25 g Essigsäure vier Stunden bei 120°C verrührt. Das in üblicher Weise isolierte Pigment wird anschliessend mit 70 g Dimethylformamid zwei Stunden bei 120°C verrührt, isoliert und in eine feinteilige Form übergeführt. Es färbt Kunststoffe und Lacke in licht- und migrationsechtem gelben Ton.

Beispiel 41: Verwendet man als Halbkondensat 1-Cyan-3'-chlorphenylcarbamylmethylen-3-iminoisoindolin und verfährt im übrigen analog Beispiel 40, so erhält man ein Kunstoffe und Lacke in grünstichig gelbem Ton färbendes Pigment.

Beispiel 42: Ersetzt man in Beispiel 40 das dort verwendete Halbkondensat durch 1-Cyan-4'-chlorphenylcarbamylmethylen-3-iminoisoindolin, so erhält man ein Pigment, das Kunststoffe und Lacke in gelbem Ton mit sehr guten Echtheitseigenschaften färbt.

Beispiel 43: Durch Umsetzung von

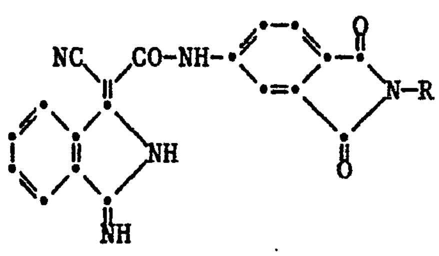

,

worin R Aethyl bedeutet, mit N-4'-Methoxyphenylbarbitursäure in Essigsäure während vier Stunden bei 112°C erhält man ein rotstichig gelb färbendes Pigment. Verwendet man das Halbkondensat obiger Formel, worin R n-Propyl bedeutet, so resultiert ein Kunststoffe und Lacke gelb färbendes Pigment.

Beispiel 44: 2,4 g 1-Cyan-N-n-propylphthalimid(4')ylcarbamylmethylen-3-iminoisoindolin und 1,56 g 1-Phenyl-3-acetylaminopyrazolon(5) werden in einem Gemisch von 60 g Essigsäure und 10 g Ameisensäure 98 % eine Stunde bei 110°C verrührt. Das entstandene dunkle Pigment wird heiss abfiltriert, mit Essigsäure sowie Aethanol gewaschen und darauf in 40 g Dimethylformamid bei 120°C 15 Min. verrührt. Man lässt die Lösung auf 20°C abkühlen, filtriert das ausgefallene Pigment ab und wäscht es mit Dimethylformamid sowie Methanol. Nach Ueberführung in eine feinteilige Form färbt das Pigment Kunststoffe und Lacke in braunem Ton von sehr guter Migrations- und guter Lichtechtheit.

Beispiel 45: Die Mischung von 2,8 g 1-Cyan-1',2',4'-triazolyl-(3')-carbamylmethylen-3-iminoisoindolin, 2,92 g 4-Cyanacetylamino-N-methylphthalimid, 60 g Essigsäure und 10 g Ameisensäure 98 % wird vier Stunden bei 110°C verrührt. Das entstandene Pigment wird heiss abfiltriert, mit Essigsäure sowie Aethanol gewaschen und mit 70 g Dimethylformamid zwei Stunden bei 120°C gerührt. Das Pigment wird abfiltriert, mit Dimethylformamid sowie Methanol gewaschen und getrocknet. Nach Ueberführung in eine feinteilige Form erhält man ein Pigment, das Kunststoffe und Lacke in gelbem Ton mit guten Echtheitseigenschaften färbt.

Beispiel 46: 2,25 g 1-[Cyan-(4'methylen-N-phthalimid)-phenylcarbamylmethylen]-3-isoindolin und 1,4 g 4-Cyanacetylaminophthalimid werden in 60 g Essigsäure unter Rühren vier Stunden auf 115°C erhitzt. Das entstandene Pigment wird abfiltriert, mit Essigsäure sowie Aethanol gewaschen und darauf mit 60 g Dimethylformamid 2 Stunden bei 120°C verrührt. Das isolierte Pigment färbt nach Ueberführung in eine feinteilige Form Kunststoffe und Lacke in rotstichig gelbem Ton und zeigt gute Echtheitseigenschaften.

Beispiel 47: Verwendet man 1,5 g 4-Cyanacetylamino-N-methylphthalimid und verfährt im übrigen analog Beispiel 46, so erhält man ein Pigment, das in seinen Eigenschaften jenem gemäss Beispiel 46 sehr ähnlich ist.

Beispiel 48: 2,25 g 1-[Cyan-(4'-methylen-N-phthalimid)-phenylcarbamylmethylen]-3-isoindolin und 1,4 g 3-Cyanacetylaminophthalimid werden in 60 g Essigsäure vier Stunden bei 115°C verrührt. Die Aufarbeitung gemäss Beispiel 46 liefert ein Pigment, das Kunststoffe und Lacke in rotstichig gelbem Ton mit guten Echtheitseigenschaften färbt. Es entspricht der Formel

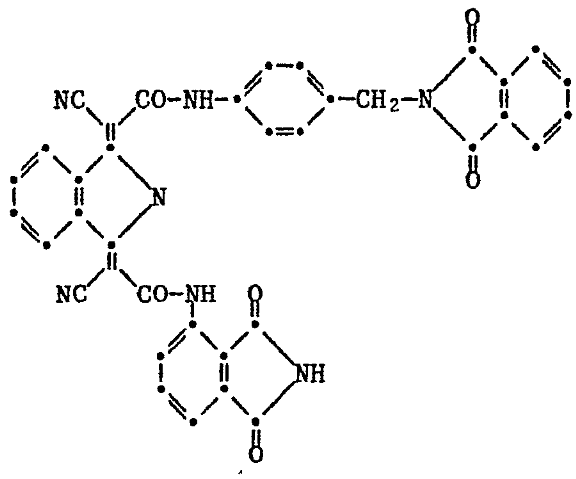

Beispiel 49: 3,3 g 1-Cyan-N-n-butylphthalimid-(4'-)ylcarbamylmethylen-3-iminoisoindolin und 2,85 g 3-Cyanacetylamino-4-chlorbenzamid werden in 40 g Essigsäure während 4 Stunden bei 110-115°C verrührt. Das bei 90°C abfiltrierte Reaktionsprodukt wird nach Waschen mit Essigsäure und Methanol mit 40 g Dimethylformamid während einer Stunde bei 90°C verrührt. Nach üblicher Aufarbeitung und Ueberführung in eine feinteilige Form erhält man ein Pigment, das PVC und Lacke in reinem grünstichig gelben Ton färbt. Die PVC-Färbung zeigt eine sehr gute Migrationsbeständigkeit. Es entspricht der Formel:

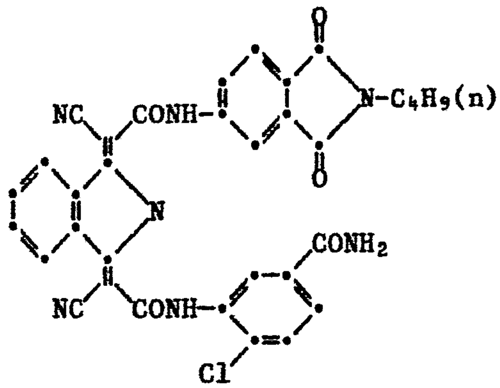

Beispiel 50: 3,1 g 1-Cyan-N-n-butylphthalimid-(4'-)ylcarbamylmethylen-3-iminoisoindolin und 3,0 g 2-Chlor-4-cyanacetylaminobenzoesäureäthylester werden in 40 g Essigsäure während 4 Stunden bei 110-115°C verrührt. Der Niederschlag wird bei 90°C abfiltriert und nach Waschen mit Essigsäure und Methanol während 2 Stunden mit 40 g Dimethylformamid bei 120°C verrührt. Das gereinigte Pigment wird bei 100°C abfiltriert, mit Dimethylformamid sowie Methanol gewaschen. Nach Trocknung und Ueberführung in eine feinteilige Form erhält man das Pigment der Formel

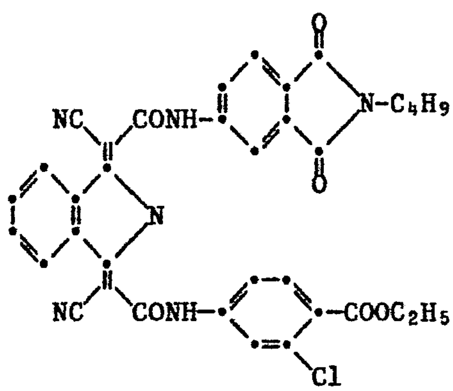

Es färbt PVC und Lacke in gelbem Ton.

Beispiel 51: 2,6 g 1-Cyan-4'carbomethoxyphenylcarbamylmethylen-3-iminoisoindolin und 2,3 g 4-Cyanacetylaminophthalimid werden in 40 g Essigsäure während 4 Stunden bei 110-115°C verrührt. Das entstandene Pigment wird bei 90°C abfiltriert, mit Essigsäure sowie Methanol gewaschen und getrocknet. Man erhält 3,3 g des Pigmentes der Formel:

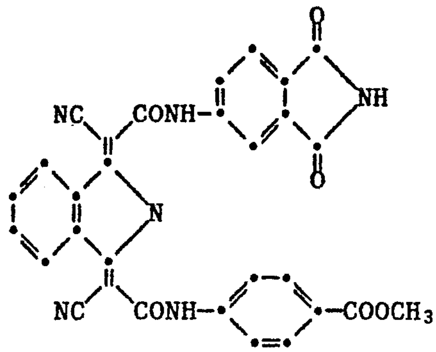

Das Pigment färbt nach Ueberführung in eine feinteilige Form Kunststoffe und Lacke in rotstichig gelbem Ton.

Beispiel 52: 3,3 g 1-Cyan-N-n-butylphthalimid-(4'-)ylcarbamylmethylen-3-iminoisoindolin und 3,0 g 1-Cyanacetylamino-2-chlor-5-trifluormethylbenzol werden in 40 g Essigsäure 4 Stunden bei 110-115°C verrührt. Der Niederschlag wird bei 90°C abfiltriert, mit Essigsäure sowie Methanol gewaschen, alsdann mit 80 g Dimethylformamid 2 Stunden bei 80°C verrührt. Das bei 80°C abfiltrierte und in üblicher Weise gewaschene und getrocknete Pigment färbt nach Ueberführung in eine feinteilige Form PVC und Lacke in grünstichig gelbem Ton. Es entspricht der Formel:

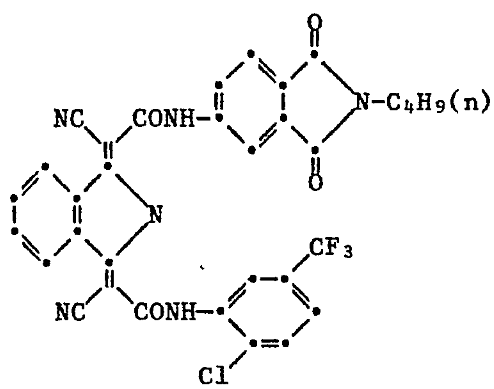

0185618

Patentansprüche

1. Isoindoline der Formel I

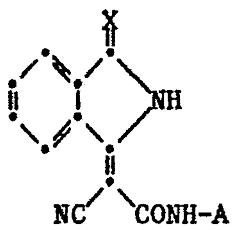 (I),

worin A für eine Gruppe der Formel II

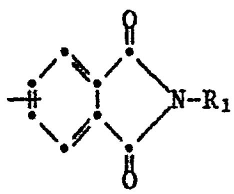 (II) steht,

worin $R_1$ -H, $C_1$-$C_4$-Alkyl oder einen unsubstituierten oder durch nicht löslichmachende Gruppen substituierten Phenylrest bedeutet, X eine Gruppe der Formel III

$$NC-\overset{\|}{C}-CONHR_2 \qquad (III)$$

worin $R_2$ einen unsubstituierten oder durch nichtlöslichmachende Substituenten substituierten isocyclischen oder einen heterocyclischen aromatischen Rest bedeutet, oder X ein heterocyclischer Rest, der einen ankondensierten Benzolkern aufweisen kann, ist.

2. Isoindoline gemäss Anspruch 1 der Formel IV

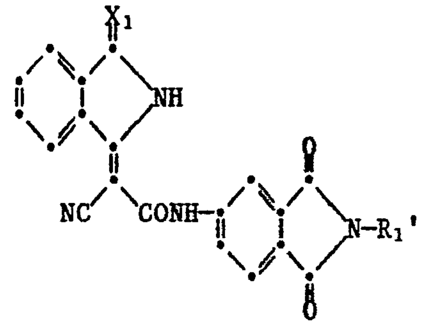

(IV),

worin $R_1$' -H oder Methyl bedeutet und $X_1$ für einen heterocyclischen Rest, ausgewählt aus der Gruppe bestehend aus den Resten der Formeln

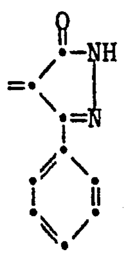

,

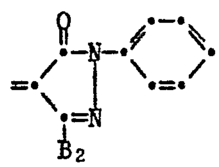

oder

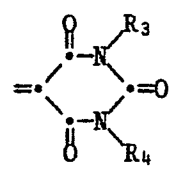

steht,

worin $B_2$ eine Gruppe $-CONH_2$ oder $-NHCOCH_3$ ist und $R_3$ und $R_4$ unabhängig voneinander -H, $C_1$-$C_4$-Alkyl, unsubstituiertes oder durch Halogen, $C_1$-$C_4$-Alkyl, $C_1$-$C_4$-Alkoxy oder Phenoxy substituiertes Phenyl bedeuten, oder $X_1$ für den Rest der Formel

$NC-\overset{\|}{C}-CONHR_5$

steht, worin $R_5$ unsubstituiertes oder durch eine oder zwei nicht löslichmachende Substituenten substituiertes Phenyl oder eine Gruppe

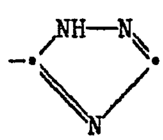

bedeutet.

0185618

3. Isoindoline gemäss Anspruch 1 der Formel V

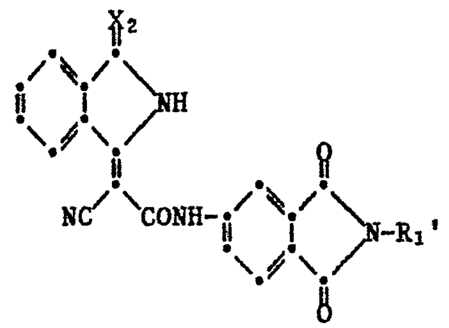

(V), worin

$X_2$ für einen Rest der Formeln

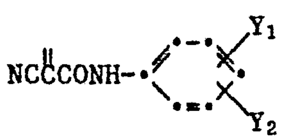

oder

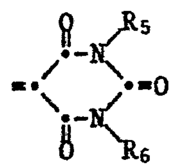

steht und $R_1$' -H oder Methyl bedeutet, $Y_1$ und $Y_2$ unabhängig voneinander ein H- oder Chloratom, eine Alkyl-, Alkoxy-, Trifluormethyl-, Carbamyl-, N-Methylcarbamyl-, Methoxycarbonyl-, Aethoxycarbonyl-, Acetylamino, unsubstituierte oder durch ein oder zwei Chloratome, Methyl- oder Methoxygruppen substituierte Benzoylaminogruppe und $R_5$ und $R_6$ unabhängig voneinander -H, $-CH_3$ oder unsubstituiertes oder durch -Cl, $-CH_3$ oder $-OCH_3$ substituiertes Phenyl bedeuten.

4. Isoindolin gemäss Anspruch 3, dadurch gekennzeichnet, dass $Y_1$ und $Y_2$ unabhängig voneinander -H, -Cl, $-CH_3$ oder $-OCH_3$ bedeuten.

0185618

5. Verfahren zur Herstellung von Isoindolinen der Formel I

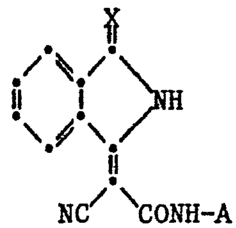

(I),

worin A für eine Gruppe der Formel II

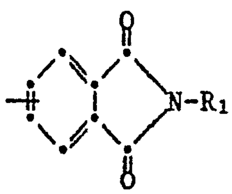

(II) steht,

worin $R_1$ -H, $C_1$-$C_4$-Alkyl oder einen unsubstituierten oder durch nichtlöslichmachende Gruppen substituierten Phenylrest bedeutet, X eine Gruppe der Formel III

$NC-\overset{\|}{C}-CONHR_2$ (III)

worin $R_2$ einen unsubstituierten oder durch nichtlöslichmachende Substituenten substituierten isocyclischen oder einen heterocyclischen aromatischen Rest bedeutet, oder X ein heterocyclischer Rest, der einen ankondensierten Benzolkern aufweisen kann, ist, dadurch gekennzeichnet, dass man in beliebiger Reihenfolge Diiminoisoindolin der Formel VI

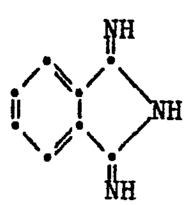

(VI)

mit je einem Mol eines Cyanacetanilides der Formel $NC-CH_2CONHA$ und der Verbindung der Formel $XH_2$ kondensiert, wobei A und X die oben in diesem Anspruch angegebene Bedeutung haben.

6. Verfahren gemäss Anspruch 5, dadurch gekennzeichnet, dass man Diiminoisoindolin mit je einem Mol eines Cyanacetamides der Formeln

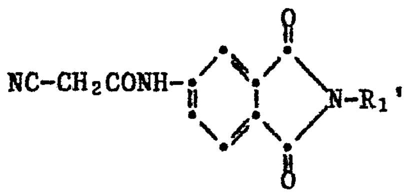

und

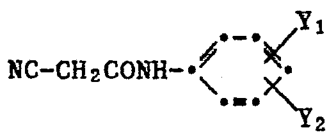

kondensiert, wobei $R_1$' -H oder Methyl, $Y_1$ und $Y_2$ unabhängig voneinander ein H- oder Chloratom, eine Alkyl-, Alkoxy-, Trifluormethyl-, Carbamyl-, N-Methylcarbamyl-, Methoxycarbonyl-, Aethoxycarbonyl-, Acetylamino, unsubstituierte oder durch ein oder zwei Chloratome, Methyl- oder Methoxygruppen substituierte Benzoylaminogruppe bedeuten.

7. Verfahren gemäss Anspruch 5, dadurch gekennzeichnet, dass man Diiminoisoindolin mit je einem Mol der Verbindungen der Formeln

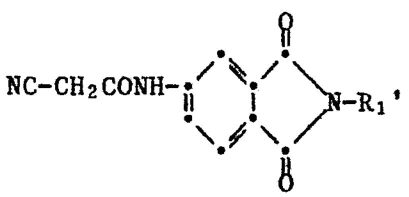

und

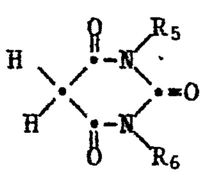

kondensiert, wobei

$R_1$' -H oder Methyl und $R_5$ und $R_6$ unabhängig voneinander -H oder unsubstituiertes oder durch -Cl, $-CH_3$ oder $-OCH_3$ substituiertes Phenyl bedeuten.

8. Verfahren zum Pigmentieren von hochmolekularem organischem Material, dadurch gekennzeichnet, dass Isoindoline der Formel I gemäss Anspruch 1 verwendet werden.

9. Hochmolekulares organisches Material enthaltend als Pigment ein Isoindolin der Formel I gemäss Anspruch 1.

FO 7.3 RU/bg*/sm*/cs*